# EUROPEAN PATENT SPECIFICATION

(11) **EP 3 454 813 B1**
(45) Date of publication and mention of the grant of the patent: **18.11.2020**
(21) Application number: 17728934.5
(22) Date of filing: 11.05.2017
(51) Int. Cl.: A61H 19/00, B29C 41/08, B29C 41/14

(54) **PROCESS FOR THE REALIZATION OF A DILDO, DILDO THUS OBTAINED AND KIT FOR ITS COMMERCIALIZATION**
VERFAHREN ZUR HERSTELLUNG EINES DILDOS, DAMIT ERHALTENER DILDO UND KIT ZU DESSEN KOMMERZIALISIERUNG
PROCÉDÉ DE RÉALISATION D'UN GODEMICHÉ, GODEMICHÉ AINSI OBTENU ET KIT POUR SA COMMERCIALISATION

(30) Priority: 13.05.2016 IT UA20163438
(43) Date of publication of application: 20.03.2019
(73) Proprietor: Ambesi, Cristian, 18038 Sanremo (IM) (IT)
(72) Inventor: Ambesi, Cristian, 18038 Sanremo (IM) (IT)
(74) Representative: Brunacci, Marco
(86) International application number: PCT/IB2017/052769
(87) International publication number: WO 2017/195146

(56) References cited:
- EP-A1- 2 436 359
- EP-A2- 2 965 739
- WO-A1-2012/135925
- DE-A1-102012 001 865

## Description

### Technical Field

The present invention relates to a process for the realization of a dildo, dildo thus obtained and kit for its commercialization.

### Background Art

Dildos are a category of objects used for erotic practices, usually shaped so as to reproduce, at least vaguely, the shape of the male genital organs.

Both because of their function, and because of their aesthetic shape, dildos are very showy objects and not very practical to be carried around.

This showiness is the cause of embarrassment for possible buyers who, often, feel the need to be able to buy the object with the utmost discretion and privacy. Furthermore, traditional dildos are substantially rigid bodies or in any case have a well-defined shape and weight.

Such characteristic makes these objects not very practical inasmuch as inconvenient to carry around and put away.

As regards carrying them around in fact, they have to be placed in special bags, or paper bags, the capacity of which must be such as to accommodate the object without its being highlighted.

A similar consideration can be made for housing dildos whenever these are not being used.

It is often in fact not very pleasant, if not embarrassing, to find objects of this type in home environments.

Their storage, therefore, requires special containers, drawers, shelves or whatever, so that the dildos can be placed in them and kept hidden.

Despite this, given their unmistakable shape, the risk remains that they can be accidentally found.

Furthermore, the drawbacks described above represent a limit for the market of these objects and in fact represent a disincentive for purchase.

It is in fact known that the users involved would like to be able to make use of a disposable object, which can be immediately discarded after use.

This not only because of aspects regarding common decency but also of hygienic aspects tied to the formation of bacteria and other pathogenic agents which can build up on the surface of the dildo and be transmitted through the repeated use of the latter over time.

EP2965739A2 discloses an inconspicuous device which is to be used as a dildo. The dildo can be filled with liquids, gels, or foams, to add a feature (e.g. heat, stiffness) or to expand the device into its wanted size.

DE102012001865A1 discloses a dildo which is packed in a box and which can be expanded by the actuation of an integrated foam cartridge.

### Description of the Invention

The main aim of the present invention is to provide a process for the realization of a dildo, a dildo thus obtained and a kit for its commercialization which provides a practical dildo to use and discreet to commercialize.

One object of the present invention is to provide a process for the realization of a dildo, a dildo thus obtained and a kit for its commercialization whose dildo is of a variable shape.

A further object of the present invention is to provide a process for the realization of a dildo, a dildo thus obtained and a kit for its commercialization whose dildo is disposable.

Another object of the present invention is to provide a process for the realization of a dildo, a dildo thus obtained and a kit for its commercialization which allow to overcome the mentioned drawbacks of the prior art within the ambit of a simple, rational, easy, effective to use and low cost solution.

The above mentioned objects are achieved by the present realization process having the characteristics of claim 1.

The above mentioned objects are also achieved by the present dildo having the characteristics of claim 9.

The above mentioned objects are then achieved by the present kit for the commercialization having the characteristics of claim 14.

### Brief Description of the Drawings

Other characteristics and advantages of the present invention will become more evident from the description of two preferred but not exclusive, embodiments of a process for the realization of a dildo, a dildo thus obtained and a kit for its commercialization illustrated by way of an indicative, but non-limiting example in the accompanying drawings, wherein:
Figures 1 and 2 are sectional views of two dildo configurations according to the invention;
Figures 3 and 4 are axonometric views of a kit for the commercialization of the dildo according to the invention;
Figure 5 is a schematic view of a first embodiment of a process for the production of a dildo according to the invention;
Figure 6 is a schematic view of a second embodiment of a process for the production of a dildo according to the invention.

### Embodiments of the Invention

With particular reference to such figures, reference numeral 1 globally designates a dildo.

The dildo 1 can be made in bright colors, but solutions cannot be ruled out involving the use of glitter, particular color combinations, monochromatic solutions or theme representations.

The dildo comprises a contact portion 2 adapted to be placed in contact with an erogenous area of a user.

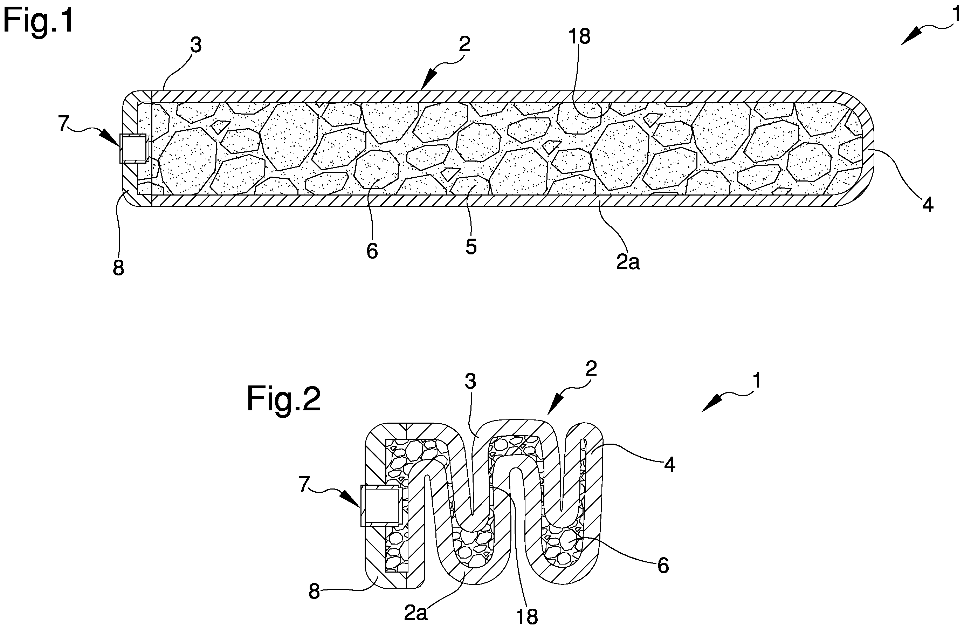

As indicated in the Figures 1 and 2, the contact portion 2 has a proximal end 3 and a distal end 4.

The proximal end 3 is the end which, during normal use of the dildo 1, is gripped by the user for the maneuverability of the object.

The distal end 4, on the other hand, is the opposite end to that described above and, in the present embodiments, has a rounded shape in order to facilitate its insertion in erogenous areas or the like.

Different shapes to those illustrated cannot be ruled out, e.g., shapes having undulated extremities, or protruding bodies, or the like, just as shapes cannot be ruled out of the entire dildo 1 different to those illustrated and having, for example, veins, protruding bodies, concavities and other details which extend along the entire surface or part thereof.

According to the invention, the contact portion 2 comprises a flexible wall 2a defining an inner chamber 18.

In particular, in a closing configuration (Figure 2) closing configuration the flexible wall 2a is folded on itself and the distal end 4 is moved close to the proximal end 3.

In an opening configuration (Figure 1) the flexible wall 2a is rigidly extended and the distal end 4 is moved away from the proximal end 3.

In this treatise, by the locution "rigidly extended" is meant a state able to maintain its shape in the absence of stresses caused by external bodies (manipulations or other mechanical forces) or able to return to its shape following the application of one or more stresses caused by external bodies (e.g., like an elastically deformable rubbery body).

Advantageously, the flexible wall 2a comprises flexible polymeric material.

In particular, the flexible polymeric material comprises at least one of plastisol and heat-treated thermoplastic powders such as, e.g., RISLAN®, or ABCITE®, or PLASCOAT®.

Advantageously, the flexible wall 2a has a thickness of between 0.5 mm and 4 mm.

The use of the materials described above in fact permits obtaining a thickness for the flexible wall 2a which is at the same time extremely reduced and extremely resistant.

This way it is possible to obtain a dildo 1 foldable in a little space without compromising its functionality.

Advantageously, the inner chamber 18 is fillable with a filling material 5 for moving from the closing configuration to the opening configuration.

In particular, the inner chamber 18 is fillable with a filling material 5, the insertion of which causes both its volumetric increase, useful to move from the first configuration to the second configuration, and an increase in the stiffness and consistency of the contact portion 2.

Advantageously, the inner chamber 18 contains sodium polyacrylate 6 adapted to interact with the filling material 5 to increase the stiffness of the contact portion 2.

Usefully, the filling material 5 comprises water.

This way, the water inside the contact portion 2 interacts with the sodium polyacrylate 6, producing a consistent gel adapted to expand the contact portion 2 from the closing configuration to the opening configuration and adapted to give the dildo 1 the stiffness and consistency necessary to make it usable for the purposes for which it is normally intended.

Different solutions cannot however be ruled out that provide for the use of different materials.

For example, the use cannot be ruled out of substances other than water as a filling material 5, such as other liquid substances or viscous substances or mixtures of various type.

The use cannot also be ruled out of substances other than sodium polyacrylate, but which have similar properties, that is, which are able to interact with the filling material 5 for the formation of a gel adapted to increase the volume, the stiffness and the consistency of the dildo 1.

The solution cannot furthermore be ruled out whereby the contact portion 2 is empty and the increase in volume, rigidity and consistency is due to the insertion of filling material 5 only.

Advantageously, the dildo 1 comprises an adjustment element 7 adapted to control the introduction of the filling material 5 in the contact portion 2.

The adjustment element 7 is preferably of the type of a retention valve, so as to allow the introduction of the filling material 5 without the latter having the chance to come out.

The adjustment element 7, furthermore, is obtained at the proximal end 3, but positioning other than that shown in the illustrations cannot be ruled out. Different solutions cannot furthermore be ruled out whereby, for example, the adjustment element 7 is of different type, e.g., a screwing/unscrewing device or a hermetically sealed cap, or other closing device.

Solutions cannot also be ruled out that provide for a different number of adjustment elements 7.

In the present embodiments, the dildo 1 comprises a sustaining portion 8 associated with the proximal end 3 and adapted to support the contact portion 2 in position.

In particular, the sustaining portion 8 is of the type of a cup sealed to the proximal end 3 so as to form a monolithic body for the dildo 1.

The sustaining portion 8 is substantially rigid and performs the function of a base for the contact portion 2.

Furthermore, the adjustment element 7 is arranged on the sustaining portion 8 so as to exploit the rigidity of the latter and avoid yielding points or points susceptible to breakage due to the difference in material.

Solutions cannot be ruled out wherein the sustaining portion 8 is not provided. According to a first embodiment, the polymer material is of the plastisol type, with hardness, assessed according to the "Shore" scale, between 50 Shore A and 92 Shore A.

The plastisol, at the thicknesses described above, is foldable and permits closing and opening the flexible wall 2a while at the same time providing the resistance needed to ensure the functionality of the dildo 1.

In a second embodiment, the polymer material is of the plastic type deriving from heat-treated thermoplastic powders and provides the same resistance and flexibility properties as plastisol.

Embodiments cannot be ruled out which envisage the use of both materials, just as embodiments cannot be ruled out wherein there is the combination between one of the materials described above and other complementary materials such as, e.g., textile and/or vegetable fibers, preformed plastics, metals, glass or the like.

In a third embodiment, for simplicity not shown in the figure, the dildo 1 comprises capsules containing expanding material, inserted into the chamber 18 and adapted to be broken for the release of the expanding material in order to obtain the opening configuration.

The above capsules contain, in particular, powdered and/or liquid material which, once having come out of the broken capsules, reacts chemically, producing a gas which, when it expands, increases the pressure inside the chamber 18, stiffening the contact portion 2 and bringing the dildo to the opening configuration.

In this third embodiment, the adjustment element 7 is not provided, since it is not necessary.

In any case, the dildo 1 is insertable in a special kit 9 for the commercialization of the dildo itself.

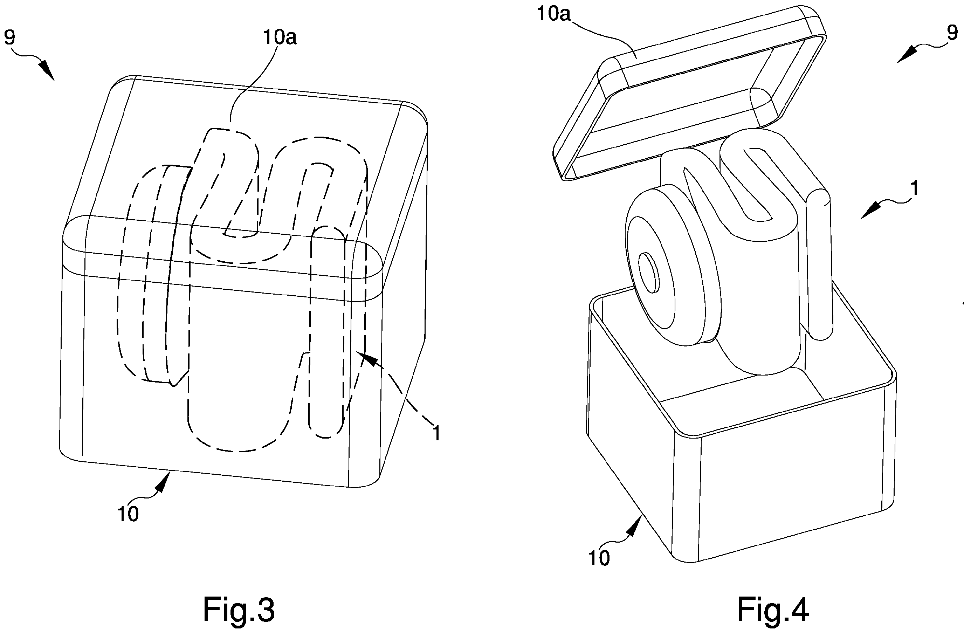

The kit 9, illustrated in the Figures 3 and 4, comprises at least a box-shaped element 10, provided with a special lid 10a, and at least a dildo 1 in the closing configuration inserted in the box-shaped element itself.

The box-shaped element 10 shown has a parallelepiped shape with square base, with height between 10 mm and 80 mm and base sides between 50 mm and 80 mm.

The box-shaped element 10 shown contains a dildo 1, but the solution cannot be ruled out wherein, according to the chosen size, the kit 9 may comprise three dildos 1 inserted into a single box-shaped element 10.

The process for the realization of a dildo 1 is as follows.

According to the invention, the process comprises a coating step A of a support body 11 with plastic material 12 for the formation of a coated body 13 comprising a contact layer 14 enveloping the support body 11. Advantageously, the support body 11 has a shape similar to the final shape to be taken by the dildo 1, i.e. it has a proximal end 3 and a distal end 4. Subsequently, the process comprises a heat treatment step B of the coated body 13 for the stabilization of the contact layer 14.

The contact layer 14 which forms on the surface of the support body 11, in fact, requires being chemically and physically stabilized to acquire the desired thickness and hardness.

Advantageously, the heat treatment step B comprises heating the coated body 13 at a temperature between 130°C and 190°C.

Usefully, heating has a duration of between 5 and 10 minutes.

Heating temperature and time values cannot be ruled out other than those indicated above, and which vary according to the degree of hardness or thickness to be given to the contact layer 14.

Still according to the invention, the process comprises a separation step C of the contact layer 14 from the support body 11 to obtain a contact portion 2 of a dildo 1.

Preferably, the separation step C comprises the mechanical separation of the contact layer 14 from the support body 11.

The contact layer 14, by now stabilized and clinging to the support body 11, is removed from the support body 11 so as to obtain what will be the contact portion 2 of the dildo 1.

Still according to the invention, the process comprises a closing step D of the contact portion 2 for the realization of the dildo 1.

In particular, the closing step D allows defining the final shape of the dildo 1, by closing the parts remained open after the separation step C.

In the present embodiments, the contact portion 2 removed from the support body 11 is open at the proximal end 3 and the closing step D provides for the closing of such end.

In particular, the process comprises a sealing step of the contact portion 2 with a support body 11.

As shown in the illustrations, the support body 11 is of the type of a rigid cup which is sealed to the contact portion 2 at the proximal end 3 so as to form a single body piece which can be filled with filling material 5.

Usefully, the sealing is done using specific glues for polymer materials such as PVC, or by means of ultrasounds.

Solutions cannot be ruled out which provide for different sealing methods, e.g., deriving from further heat treatments of the parts to be sealed.

According to a first and a second embodiment, the process comprises, prior to the sealing step, a filling step of the contact portion 2 with sodium polyacrylate, for simplicity not shown in the figures.

This way, the dildo 1 is chemically charged, i.e., prepared to receive subsequent filling material 5 with which the sodium polycrylate can give rise to gelification chemical reactions inside the contact portion 2 so that the dildo 1 moves from a closing configuration to an opening configuration.

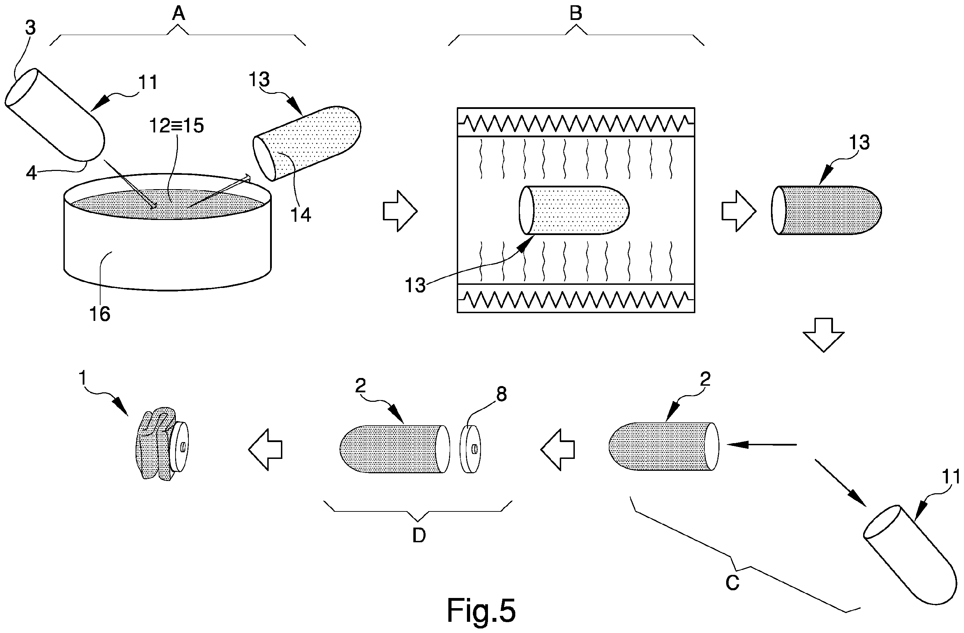

According to a first embodiment, shown in Figure 5, the coating step A comprises covering the support body 11 with liquid plastisol 15 for the formation of the coated body 13 comprising the contact layer 14 enveloping the support body 11.

Usefully, the process according to the first embodiment comprises a preheating step of the plastic material 12 at a temperature between 150°C and 220°C. Preferably, the liquid and preheated plastisol 15 is contained in a special container 16 and the support body 11 is soaked in this container.

Different embodiments cannot be ruled out wherein, for example, the plastisol 15 is made to cast onto the support body 11.

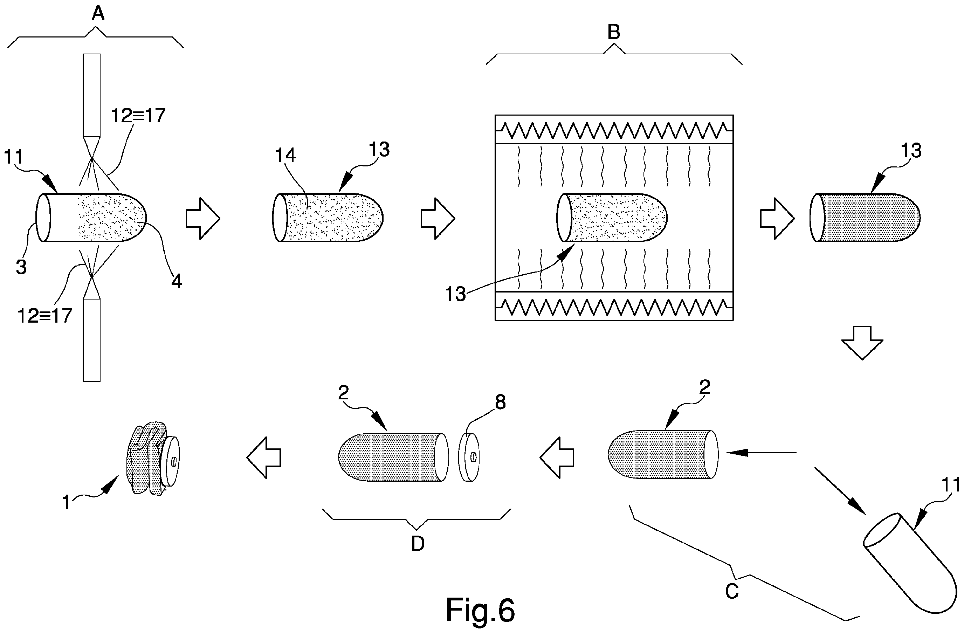

In a second embodiment, shown in Figure 6, the coating step A comprises covering the support body 11 with thermoplastic powders 17 for the formation of the coated body 13 comprising the contact layer 14 enveloping the support body itself.

Preferably, the thermoplastic powders 17 are of the RISLAN®, or ABCITE®, or PLASCOAT® type, but other thermoplastic powders cannot be ruled out having the same chemical and molecular structures as those described, or different thermoplastic powders, but which are able to form a contact layer 14 like that described above.

According to a third embodiment, not shown in the illustrations, the process comprises, prior to the sealing step, a filling step of the contact portion 2 with insertable expanding material in the form of capsules.

It has in practice been ascertained that the described invention achieves the intended objects and in particular the fact is underlined that the devised process allows obtaining a dildo movable between a closing configuration and an opening configuration in such a way as to be used and commercialized in a practical and discreet way.

The process in fact permits obtaining a contact portion having such thicknesses to allow the folding of the same, but at the same time able to provide material resistance values to permit its stiffening and use as an object for erotic practices. The devised dildo, furthermore, is particularly suitable for "disposable" use inasmuch as the material used, the operating modes and the costs allow the user to discard the dildo immediately after use, with consequent benefits tied to hygiene and practicality.

## Claims

1. Process for the realization of a dildo, comprising:
- a coating step (A) of a support body (11) with plastic material (12) for the formation of a coated body (13) comprising a contact layer (14) enveloping said support body (11);
- a heat treatment step (B) of said coated body (13) for the stabilization of said contact layer (14);
- a separation step (C) of said contact layer (14) from said support body (11) to obtain a contact portion (2) of a dildo (1);
- a closing step of said contact portion (2) for the realization of said dildo (1); **characterized in that** it comprises, prior to said sealing step, a filling step of said contact portion (2) with insertable expanding material in the form of capsules or a filling step of said contact portion (2) with sodium polyacrylate (6).

2. Process according to claim 1, **characterized by** the fact that said coating step (A) comprises covering a support body (11) with liquid plastisol (15) for the formation of a coated body (13) comprising a contact layer (14) enveloping said support body (11).

3. Process according to claim 1, **characterized by** the fact that said coating step (A) comprises covering a support body (11) with thermoplastic powders (17) for the formation of a coated body (13) comprising a contact layer (14) enveloping said support body (11).

4. Process according to one or more of the preceding claims, **characterized by** the fact that it comprises a preheating step of said plastic material (12) at a temperature between 150°C and 220°C.

5. Process according to one or more of the preceding claims, **characterized by** the fact that said heat treatment step (B) comprises heating said coated body (13) at a temperature between 130°C and 190°C.

6. Process according to one or more of the preceding claims, **characterized by** the fact that said heating has a duration of between 5 and 10 minutes.

7. Process according to one or more of the preceding claims, **characterized by** the fact that said separation step (C) comprises the mechanical separation of said contact layer (14) from said support body (11).

8. Process according to one or more of the preceding claims, **characterized by** the fact that it comprises a sealing step of said contact portion (2) with a sustaining portion (8).

9. Dildo (1) comprising a contact portion (2) adapted to be placed in contact with an erogenous area of a user and having a proximal end (3) and a distal end (4), wherein said contact portion (2) comprises at least in part a flexible wall (2a) defining an inner chamber (18) and wherein:
- in a closing configuration said flexible wall (2a) is folded on itself and said distal end (4) is moved close to said proximal end (3);
- in an opening configuration said flexible wall (2a) is rigidly extended and said distal end (4) is moved away from said proximal end (3);
**characterized in that** said inner chamber (18) is either fillable with a filling material (5) and contains sodium polyacrylate (6) adapted to interact with said filling material (5) to increase the stiffness of the contact portion (2) or it contains capsules containing expanding material, inserted in said chamber (18) and adapted to be broken to release said expanding material to obtain said opening configuration.

10. Dildo (1) according to one or more of claim 9, **characterized by** the fact that said flexible wall (2a) comprises flexible polymeric material.

11. Dildo (1) according to one or more claims 9 to 10, **characterized by** the fact that said flexible polymeric material comprises at least one of plastisol and heat-treated thermoplastic powders.

12. Dildo (1) according to one or more of claims 9 to 11, **characterized by** the fact that it comprises at least one adjustment element (7) adapted to control the introduction of at least part of said filling material (5) in said contact portion (2).

13. Dildo (1) according to one or more of claims 9 to 12, **characterized by** the fact that it comprises a sustaining portion (8) associated with said proximal end (3) and adapted to support said contact portion (2) in position.

14. Kit (9) for the commercialization of a dildo comprising:
- a box-shaped element (10);
- at least one dildo (1) according to one or more of claims 9 to 13, in the closing configuration and inserted in said box-shaped element (10).

## Patentansprüche

1. Verfahren zur Realisierung eines Dildos, umfassend:
- einen Beschichtungsschritt (A) eines Trägerkörpers (11) mit Kunststoffmaterial (12) zur Bildung eines beschichteten Körpers (13) mit einer Kontaktschicht (14), die den Trägerkörper (11) umhüllt;
- einen Wärmebehandlungsschritt (B) des beschichteten Körpers (13) zur Stabilisierung der Kontaktschicht (14);
- einen Trennungsschritt (C) der Kontaktschicht (14) von dem Trägerkörper (11), um einen Kontaktabschnitt (2) eines Dildos (1) zu erhalten;
- einen Schließschritt des Kontaktabschnitts (2) zur Realisierung des Dildos (1);
**dadurch gekennzeichnet, dass** es vor dem Versiegelungsschritt einen Befüllungsschritt des Kontaktabschnitts (2) mit einsetzbarem expandierendem Material in Form von Kapseln oder einen Befüllungsschritt des Kontaktabschnitts (2) mit Natriumpolyacrylat (6) umfasst.

2. Verfahren nach Anspruch 1, **dadurch gekennzeichnet, dass** der Beschichtungsschritt (A) das Beschichten eines Trägerkörpers (11) mit flüssigem Plastisol (15) zur Bildung eines beschichteten Körpers (13) umfasst, der eine Kontaktschicht (14) aufweist, die den Trägerkörper (11) umhüllt.

3. Verfahren nach Anspruch 1, **dadurch gekennzeichnet, dass** der Beschichtungsschritt (A) das Beschichten eines Trägerkörpers (11) mit thermoplastischen Pulvern (17) zur Bildung eines beschichteten Körpers (13) umfasst, der eine Kontaktschicht (14) aufweist, die den Trägerkörper (11) umhüllt.

4. Verfahren nach einem oder mehreren der vorstehenden Ansprüche, **dadurch gekennzeichnet, dass** es einen Vorheizschritt des Kunststoffmaterials (12) bei einer Temperatur zwischen 150°C und 220°C umfasst.

5. Verfahren nach einem oder mehreren der vorhergehenden Ansprüche, **dadurch gekennzeichnet, dass** der Wärmebehandlungsschritt (B) das Erwärmen des beschichteten Körpers (13) auf eine Temperatur zwischen 130°C und 190°C umfasst.

6. Verfahren nach einem oder mehreren der vorhergehenden Ansprüche, **dadurch gekennzeichnet, dass** das Erwärmen eine Dauer zwischen 5 und 10 Minuten hat.

7. Verfahren nach einem oder mehreren der vorhergehenden Ansprüche, **dadurch gekennzeichnet, dass** der Trennungsschritt (C) die mechanische Trennung der Kontaktschicht (14) von dem Trägerkörper (11) umfasst.

8. Verfahren nach einem oder mehreren der vorhergehenden Ansprüche, **dadurch gekennzeichnet, dass** es einen Versiegelungsschritt des Kontaktabschnitts (2) mit einem Stützabschnitt (8) umfasst.

9. Dildo (1), umfassend einen Kontaktabschnitt (2), der ausgebildet ist, mit einer erogenen Zone eines Benutzers in Kontakt gebracht zu werden, und der ein proximales Ende (3) und ein distales Ende (4) aufweist, wobei der Kontaktabschnitt (2) zumindest teilweise eine flexible Wand (2a) umfasst, die eine innere Kammer (18) definiert, und wobei:
- in einer Schließkonfiguration die flexible Wand (2a) auf sich selbst gefaltet ist und das distale Ende (4) nahe an das proximale Ende (3) bewegt ist;
- in einer Öffnungskonfiguration die flexible Wand (2a) starr verlängert ist und das distale Ende (4) von dem proximalen Ende (3) wegbewegt ist;
**dadurch gekennzeichnet, dass** die innere Kammer (18) entweder mit einem Füllmaterial (5) füllbar ist und Natriumpolyacrylat (6) enthält, das ausgebildet ist, mit dem Füllmaterial (5) zusammenzuwirken, um die Steifigkeit des Kontaktabschnitts (2) zu erhöhen, oder sie Kapseln enthält, die expandierendes Material enthalten, das in die Kammer (18) eingesetzt ist und ausgebildet ist, gebrochen zu werden, um das expandierende Material freizugeben, um die Öffnungskonfiguration zu erhalten.

10. Dildo (1) nach Anspruch 9, **gekennzeichnet durch** die Tatsache, dass die flexible Wand (2a) aus flexiblem Polymermaterial besteht.

11. Dildo (1) nach einem oder mehreren Ansprüchen 9 bis 10, **dadurch gekennzeichnet, dass** das flexible Polymermaterial mindestens eines von Plastisol und wärmebehandelten thermoplastischen Pulvern enthält.

12. Dildo (1) nach einem oder mehreren der Ansprüche 9 bis 11, **dadurch gekennzeichnet, dass** er mindestens ein Einstellelement (7) aufweist, das ausgebildet ist, die Einführung mindestens eines Teils des Füllmaterials (5) in den Kontaktbereich (2) zu steuern.

13. Dildo (1) nach einem oder mehreren der Ansprüche 9 bis 12, **dadurch gekennzeichnet, dass** er einen Stützabschnitt (8) aufweist, der mit dem proximalen Ende (3) verbunden und ausgebildet ist, den Kontaktabschnitt (2) in Position zu halten.

14. Kit (9) für die Vermarktung eines Dildos, umfassend:
- ein kastenförmiges Element (10);
- mindestens einen Dildo (1) nach einem oder mehreren der Ansprüche 9 bis 13 in der Schließkonfiguration, der in das kastenförmige Element (10) eingesetzt ist.

## Revendications

1. Procédé de réalisation d'un godemiché, comprenant :
- une étape de revêtement (A) d'un corps de support (11) avec une matière plastique (12) pour la formation d'un corps revêtu (13) comprenant une couche de contact (14) enveloppant ledit corps de support (11) ;
- une étape de traitement thermique (B) dudit corps revêtu (13) pour la stabilisation de ladite couche de contact (14) ;
- une étape de séparation (C) de ladite couche de contact (14) depuis ledit corps de support (11) pour obtenir une portion de contact (2) d'un godemiché (1) ;
- une étape de fermeture de ladite portion de contact (2) pour la réalisation dudit godemiché (1) ;
**caractérisé en ce qu'**il comprend, avant ladite étape de scellement, une étape de remplissage de ladite portion de contact (2) avec une matière expansible insérable sous la forme de capsules ou une étape de remplissage de ladite portion de contact (2) avec du polyacrylate de sodium (6).

2. Procédé selon la revendication 1, **caractérisé par le fait que** ladite étape de revêtement (A) comprend le recouvrement d'un corps de support (11) avec un plastisol liquide (15) pour la formation d'un corps revêtu (13) comprenant une couche de contact (14) enveloppant ledit corps de support (11).

3. Procédé selon la revendication 1, **caractérisé par le fait que** ladite étape de revêtement (A) comprend le recouvrement d'un corps de support (11) avec des poudres thermoplastiques (17) pour la formation d'un corps revêtu (13) comprenant une couche de contact (14) enveloppant ledit corps de support (11).

4. Procédé selon une ou plusieurs des revendications précédentes, **caractérisé par le fait qu'**il comprend une étape de préchauffage de ladite matière plastique (12) à une température entre 150 °C et 220 °C.

5. Procédé selon une ou plusieurs des revendications précédentes, **caractérisé par le fait que** ladite étape de traitement thermique (B) comprend le chauffage dudit corps revêtu (13) à une température entre 130 °C et 190 °C.

6. Procédé selon une ou plusieurs des revendications précédentes, **caractérisé par le fait que** ledit chauffage a une durée entre 5 et 10 minutes.

7. Procédé selon une ou plusieurs des revendications précédentes, **caractérisé par le fait que** ladite étape de séparation (C) comprend la séparation mécanique de ladite couche de contact (14) depuis ledit corps de support (11).

8. Procédé selon une ou plusieurs des revendications précédentes, **caractérisé par le fait qu'**il comprend une étape de scellement de ladite portion de contact (2) avec une portion de soutien (8).

9. Godemiché (1) comprenant une portion de contact (2) apte à être placée en contact avec une zone érogène d'un utilisateur et ayant une extrémité proximale (3) et une extrémité distale (4), dans lequel ladite portion de contact (2) comprend au moins en partie une paroi flexible (2a) définissant une chambre intérieure (18) et dans lequel :
- dans une configuration de fermeture, ladite paroi flexible (2a) est repliée sur elle-même et ladite extrémité distale (4) est rapprochée de ladite extrémité proximale (3) ;
- dans une configuration d'ouverture, ladite paroi flexible (2a) est rigidement étendue et ladite extrémité distale (4) est éloignée de ladite extrémité proximale (3) ; **caractérisé en ce que** soit ladite chambre intérieure (18) est en mesure d'être remplie avec une matière de remplissage (5) et contient du polyacrylate de sodium (6) apte à interagir avec ladite matière de remplissage (5) pour augmenter la rigidité de la portion de contact (2) soit elle contient des capsules contenant une matière expansible, insérées dans ladite chambre (18) et aptes à être cassées pour libérer ladite matière expansible afin d'obtenir ladite configuration d'ouverture.

10. Godemiché (1) selon l'une ou plusieurs de la revendication 9, **caractérisé par le fait que** ladite paroi flexible (2a) comprend une matière polymère flexible.

11. Godemiché (1) selon une ou plusieurs des revendications 9 à 10, **caractérisé par le fait que** ladite matière polymère flexible comprend au moins l'un parmi un plastisol et des poudres thermoplastiques traitées thermiquement.

12. Godemiché (1) selon une ou plusieurs des revendications 9 à 11, **caractérisé par le fait qu'**il comprend au moins un élément d'ajustement (7) apte à commander l'introduction d'au moins une partie de ladite matière de remplissage (5) dans ladite portion de contact (2).

13. Godemiché (1) selon une ou plusieurs des revendications 9 à 12, **caractérisé par le fait qu'**il comprend une portion de soutien (8) associée à ladite extrémité proximale (3) et apte à supporter ladite portion de contact (2) en position.

14. Kit (9) pour la commercialisation d'un godemiché comprenant :
- un élément en forme de boîte (10) ;
- au moins un godemiché (1) selon une ou plusieurs des revendications 9 à 13, dans la configuration de fermeture et inséré dans ledit élément en forme de boîte (10).
